# NEUE EUROPÄISCHE PATENTSCHRIFT

(11) **EP 0 889 741 B2**
(45) Veröffentlichungstag und Bekanntmachung des Hinweises auf die Entscheidung über den Einspruch: **02.06.2010**
(45) Hinweis auf die Patenterteilung: 18.12.2002
(21) Anmeldenummer: 97915431.7
(22) Anmeldetag: 25.03.1997
(51) Int. Cl.: A61L 9/04

(54) **TRÄGERKÖRPER ZUR GEREGELTEN ABGABE FLÜCHTIGER SUBSTANZEN**
SUBSTRATE FOR THE CONTROLLED RELEASE OF VOLATILE SUBSTANCES
SUPPORT POUR LIBERATION REGULEE DE SUBSTANCES VOLATILES

(30) Priorität: 26.03.1996 DE 19611993
(43) Veröffentlichungstag der Anmeldung: 13.01.1999
(73) Patentinhaber: Rückleben, Maren, 22559 Hamburg (DE)
(72) Erfinder: ALBATH, Heidrun, Elisabeth, D-25596 Wacken (DE); RÜCKLEBEN, Maren, D-22559 Hamburg (DE)
(74) Vertreter: Hagemann, Heinrich
(86) Internationale Anmeldenummer: PCT/EP1997/001515
(87) Internationale Veröffentlichungsnummer: WO 1997/035626

(56) Entgegenhaltungen:
- FR-A- 350 406
- FR-A- 2 088 119
- FR-A- 2 611 985
- GB-A- 2 177 602
- US-A- 4 411 855
- US-A- 4 445 641
- US-A- 4 476 171
- US-A- 4 567 613
- US-A- 4 802 626

## Beschreibung

Die Erfindung bezieht sich auf einen Trägerkörper zur geregelten Abgabe flüchtiger Substanzen, wobei diese zur Desinfektion bzw. Desodorierung von Räumen oder zu ähnlichen Zwecken, die allgemein der Raumlufterfrischung oder -verbesserung dienen, eingesetzt werden.

Duft- bzw. Wirkstoffspender, die abhängig von ihrer Funktionsweise den sogenannten "Slow-Release"-Systemen oder den sogenannten "Controlled-Release"-Systemen zuzuordnen sind und im wesentlichen auf der geregelten Freisetzung eines Reservoirs von Duft- bzw. Wirkstoffen durch Nutzung verschiedener physikalischer bzw. chemischer Eigenschaften basieren, finden heutzutage nicht nur im häuslichen und hier besonders im sanitären Bereich oder im Innenraum von Fahrzeugen, sondern in zunehmendem Maße auch in der Industrie sowie in der Land- und Forstwirtschaft breite Anwendungsfelder. Durch die Speicherung und Abgabe flüchtiger Substanzen mit desinfizierender Wirkung dienen sie beispielsweise der Vernichtung von Ungeziefer. Gleichfalls werden sie zur Speicherung und Abgabe flüchtiger Substanzen mit desodorierender Funktion verwendet, was eine Erfrischung bzw. Verbesserung der Raumluft zur Folge hat.

Bislang eingesetzte Trägermaterialien haben sich hierbei insofern als nachteilig erwiesen, als die gespeicherten Substanzen durch sie im Normalfall zu schnell oder zu langsam abgegeben werden. In einer verbreiteten Variante wird beispielsweise der Duft- bzw. Wirkstoff in ein Kunststoffbehältnis eingebracht, das mit einer Cellulosemembran verschlossen wird. Diese Membran weist keine definierte Porenstruktur auf, so daß die Stoffabgabe nur schwer geregelt werden kann. Bei schwankenden Luftbewegungen kann die Membran sehr schnell austrocknen; der Nachschub an gespeichertem Stoff stagniert dann. Eine gealterte Membran weist weiterhin eine unerwünschte selektive Wirkung auf, so daß nicht mehr die gesamte Duft- bzw. Wirkstoffpalette freigesetzt wird, das heißt die Zusammensetzung der Duft- bzw. Wirkstoffe ändert sich im Laufe der Zeit.

Eine einfachere Möglichkeit zur Speicherung und Abgabe von Duft- bzw. Wirkstoffen besteht in der Verwendung poröser Körper, bei denen sich der Duft- bzw. Wirkstoff in den Poren des porösen Körpers befindet und aus diesen Poren heraus verdunstet.

Trägerkörper aus Keramik weisen nur niedrige Porenvolumina bis zu 40 % auf und verfügen nur über einen geringen Anteil offener Poren. Die zu verdampfenden Stoffe können hier überwiegend auf der äußeren Oberfläche gespeichert werden, so daß nur geringe Substratmengen pro Raumeinheit aufgebracht werden können. Ebenfalls können die in Keramiken unvermeidbar enthaltenen Mineralstoffe zu einer selektiven Fixierung von Aromastoffen führen. Die deutsche Patentschrift DE 44 17 739 beschreibt hierbei in ähnlichem Zusammenhang die Verwendung eines aus offenporigem Sinterglas bestehenden porösen Körpers zur Speicherung und geregelten Abgabe verdampfbarer Substanzen. Der Körper hat vorzugsweise ein Porenvolumen von 30 % bis 85 % und eine mittlere Porengröße von 10 µm bis 350 µm.

Für eine nachhaltige und dauerhafte Wirkung von "Slow-Release"-Systemen, bei denen eine Duft- bzw. Wirkstoffe abgebende Einheit fortwährend mit zu verflüchtigender Substanz versorgt wird, ist es von großer Bedeutung, daß die Verdunstung (= sich unterhalb des Siedepunkts einer Substanz vollziehender Übergang vom flüssigen in den gasförmigen Aggregatzustand) bzw. die Sublimation (= direkter Übergang einer Substanz vom festen in den gasförmigen Aggregatzustand, ohne daß der normalerweise dazwischenliegende flüssige Aggregatzustand angenommen wird) die Duft- bzw. Wirkstoffe kontinuierlich über einen längeren Zeitraum hinweg, das heißt je nach Einsatzgebiet mindestens einige Stunden lang, möglichst gleichmäßig mit konstanter Konzentration und mit konstanter Zusammensetzung abgibt. Hierbei ist jedoch eine gezielte Regelung der Abgabe der Duft- bzw. Wirkstoffe nur schwer, in manchen Fällen sogar überhaupt nicht möglich.

Dementsprechend weisen "Slow-Release"-Systeme diverse Nachteile und Unzulänglichkeiten auf. So sind sie im allgemeinen reaktionsträge, das heißt in geschlossenen Räumen vergeht aufgrund der langsamen Freisetzung der Wirkstoffe einige Zeit, bis die angestrebte Wirkstoffkonzentration erreicht ist. Dies steht jedoch in deutlichem Widerspruch zu dem Ziel, beispielsweise bei der Beduftung von Wohn- oder Geschäftsräumen den Duft unmittelbar nach der Anwendung wahrzunehmen. Auch bei der Anwendung von Insektiziden in geschlossenen Räumen ist es wünschenswert, die notwendige insektizide Konzentration möglichst schnell zu erzielen, um nicht mehr Wirkstoff als unbedingt notwendig einsetzen zu müssen. Anschließend soll die Wirkstoffkonzentration über einen bestimmten Zeitraum aufrechterhalten werden.

Im allgemeinen ist den "Slow-Release"-Systemen des weiteren eine gewisse Ineffizienz nicht abzusprechen, da sie die Wirkstoffe permanent verdunsten, das heißt auch in Zeiten freisetzen, in denen gar kein diesbezüglicher Bedarf besteht. Darüber hinaus wird durch die bekannten "Slow-Release"-Systeme auch den heutzutage stetig ansteigenden Anforderungen an den Umweltschutz in keinster Weise Rechnung getragen, da die "Slow-Release"-Systeme einen hohen Verpackungsaufwand erfordern und in den meisten Fällen nicht wiederauffüllbar sind.

Im Gegensatz zu den "Slow-Release"-Systemen kann bei der Verwendung von Dosiereinrichtungen wie etwa Sprays, Zerstäubersystemen, Tropfflaschen oder gegebenenfalls auch Pinselflaschen eine Portion des Duft- bzw. Wirkstoffs bei Bedarf durch Aufbringen auf eine oder mehrere Trägerseiten sofort freigesetzt werden, so daß Dosiereinrichtungen im Regelfall durch eine unmittelbare Wirkung charakterisiert sind, die bislang allerdings oft nur für eine relativ kurze Zeitspanne vorhielt.

Eine hierbei zum Zwecke der kontinuierlichen Abgabe von Duft- bzw. Wirkstoffen vielfach verwendete Technik besteht darin, aus einem Zerstäuber eine bestimmte Menge eines Duft- bzw. Wirkstoffs abzugeben und in ein Trägermaterial zu sprühen, wobei als Trägermaterial nach dem Stand der Technik Cellulosekarton, Filz oder Vlies vorgeschlagen wurden. Diese Materialien werden üblicherweise in "Slow-Release"-Systemen verwendet und saugen den Duft- bzw. Wirkstoff nahezu vollständig auf.

So offenbart die deutsche Offenlegungsschrift DE 25 40 075 eine Aerosoldose mit einem wenigstens teilweise im Weg des austretenden Sprühstrahls angebrachten Substrat aus saugfähigem, durchlässigem Material, vorzugsweise Papier- oder Pappmaterial. Obwohl hierbei die Größe der Oberfläche des Substrats, das in einer Schutzkappe bzw. in Form einer Schutzkappe der Aerosoldose ausgebildet ist, sowie die Größe der in der Schutzkappe angeordneten Durchtrittsöffnung und Perforationen einstellbar sind, entfaltet die Aerosoldose hauptsächlich im Moment der Betätigung eine kurzzeitige, dann jedoch relativ starke Wirkung. Die nachfolgende Wirkung über einen längeren Zeitraum erfolgt naturgemäß nur mit wesentlich verminderter Intensität, da der austretende Duft- bzw. Wirkstoff ziemlich rasch gebunden bzw. verbraucht wird. Während und kurz nach dem Versprühen ist zwar reichlich Aerosol bzw. Wirkstoff in der umgebenden Luft und damit eine starke Wirkung vorhanden, diese verfliegt jedoch ziemlich schnell, da beispielsweise mit der Entlüftung der Duft- bzw. Wirkstoff rasch wieder entweicht, so daß danach die die Luft verschlechternden Wirkungsquellen überwiegen.

In der deutschen Offenlegungsschrift DE 23 31 981 wird eine Betätigungskappe für Aerosol-Behälter vorgeschlagen, die, wenn sie anstelle der normalen abnehmbaren Kappe auf den Aerosol-Behälter aufgebracht wird, eine einfache Betätigung des Flüssigkeitsfreigabe-Ventils zum Versprühen der Flüssigkeit gestattet, wobei die Hand des Benutzers gegen einen Kontakt mit der Flüssigkeit geschützt ist und wobei das Versprühen in der Weise erfolgt, daß ein Teil der versprühten Flüssigkeit in der üblichen Weise frei in die Luft abgestrahlt wird, während der Rest der Flüssigkeit ein absorbierendes Material imprägniert, von dem es verdampfen kann, um auf diese Weise sowohl eine schnelle als auch eine unterstützende Wirkung für Chemikalien der Flüssigkeit zu erhalten. Die in der DE 23 31 981 offenbarte Betätigungskappe für Aerosol-Behälter ist hierbei insbesondere für die Benutzung bei Aerosol-Behältern gedacht, die ein Ventil besitzen, das den Flüssigkeitsinhalt in axialer Richtung abgibt und durch Fingerdruck betätigt werden kann.

Aus der deutschen Offenlegungsschrift DE 37 06 256 ist eine Abgabevorrichtung für flüchtige Substanzen, insbesondere Luftverbesserer bekannt, die einen Aerosol-Druckbehälter umfaßt, der die abzugebenden Substanzen zusammen mit einem Treibmittel enthält. Die Betätigung des Abgabeventils des Behälters erfolgt durch einen hohlen Druckknopf, der wenigstens einen durchbrochenen Wandbereich aufweist und der in Längsrichtung mit dem Abgabekopf zusammenwirkt, welcher mit dem beweglichen Teil des Abgabeventils verbunden ist. In dem Druckknopf ist wenigstens ein absorbierendes Element untergebracht, das mit den Substanzen imprägniert wird, die aus dem Druckbehälter durch wenigstens eine im Abgabekopf vorhandene Auslaßöffnung abgegeben werden und die in das Innere des Druckknopfes und gegen eine Ablenkplatte geschleudert werden und dann auf das absorbierende Element herabfallen.

Vorstehend genannten Druckschriften DE 25 40 075, DE 23 31 981 und DE 37 06 256 ist gemeinsam, daß jeweils aus einem Zerstäuber eine bestimmte Menge eines Duft- bzw. Wirkstoffs abgegeben und in einen Träger gesprüht wird. Als häufig eingesetzte, preiswerte Träger werden hierbei Ganzkörper aus Cellulosefasern, insbesondere Cellulosefilze, -karton oder -vliese vorgeschlagen. Diese Materialien, die üblicherweise für die eingangs beschriebenen "Slow-Release"-Systeme verwendet werden, besitzen keine definierte Porenstruktur und reduzieren durch ihre Faserstrukturen die Wirkung der Kapillarkräfte. Die zahlreichen Hydroxylgruppen der Cellulose führen durch ihre Wechselwirkung mit polaren Gruppen der Duft- bzw. Wirkstoffe und mit Wassermolekülen dazu, daß bereits bei der Speicherung eine unbeabsichtigt starke Fixierung von Wirk- und Aromastoffen erfolgen kann und daß bei der Wirkstoffabgabe unerwünschte Chromatographieeffekte auftreten, wodurch bei den meisten Wirk- und Aromastoffen bzw. Stoffgemischen mittlere bis starke Beeinträchtigungen hinsichtlich der Raumwirkung, beispielsweise Verschiebungen der Duftwirkungen im Vergleich zu nicht adsorbiertem Ursprungsmaterial, hervorgerufen werden.

Ein weiterer Nachteil der Cellulosekörper besteht in der Quellbarkeit derselben. Bei höherer Luftfeuchtigkeit kann der Cellulosekörper schichtweise aufbrechen, wobei der dann folgende übermäßige Luftzutritt die Wirkungsdauer verkürzt und die erwünschte Wirkstoffkonzentration in der Raumluft verändert. Somit kann es bei den Cellulosekörpern zu Verformungen und zu Verfärbungen kommen, was den ästhetischen Eindruck und damit natürlich auch die Verkaufsfähigkeit von Produkten mit derartigen Trägern nachhaltig beeinträchtigt.

Festzuhalten bleibt also, daß die bislang bei "Slow-Release"-Systemen eingesetzten Trägermaterialien aus Cellulosefasern bei den in den Druckschriften DE 25 40 075, DE 23 31 981 und DE 37 06 256 jeweils vorgestellten Dosiereinrichtungen nicht in gewünschter Weise funktionieren, da die Verdunstung der Duft- bzw. Wirkstoffe durch die vorgeschlagenen Trägermaterialien nicht in ordnungsgemäßer Weise geregelt wird. Die vorgeschlagenen Trägermaterialien saugen die Wirkstoffe unzweckmäßigerweise auf und verdunsten diese somit mit zu geringer Rate bzw. zu erheblichen Teilen überhaupt nicht.

Aus dem deutschen Gebrauchsmuster DE 93 18 043.8 sind flächige Duftsteine aus geschrühtem Ton in der Form eines halben Pfirsichkerns bekannt, die auf der gewölbten Oberseite mit Vertiefungen versehen sind. Diese Duftsteine werden in Wasser getaucht und mit Duftölen beträufelt, wodurch sie tagelang ihren Duft verströmen.

Die Druckschrift GB 286 862 offenbart einen flüchtigen desodorierenden oder desinfizierenden Block, der extern mit Oberflächenvertiefungen, wie beispielsweise Rillen, versehen ist, so daß die Oberfläche im Verhältnis zum Volumen wesentlich vergrößert wird. Aus der Druckschrift GB 286 862 geht jedoch nicht explizit hervor, daß die flüchtigen Substanzen auf den Trägerkörper dosierbar aufbringbar sind, da beispielsweise ein desinfizierender Block aus festem Naphthalen beschrieben wird. Des weiteren geht aus der Druckschrift GB 286 862 nicht explizit hervor, daß das Trägermaterial chemisch inert sein muß.

Festzustellen ist schließlich, daß weder im deutschen Gebrauchsmuster DE 93 18 043.8 noch in der Druckschrift GB 286 862 Angaben hinsichtlich des Verhältnisses von Oberfläche zu Volumen des Trägerkörpers gemacht werden.

Ausgehend von den vorstehend geschilderten Unzulänglichkeiten liegt der vorliegenden Erfindung die Aufgabe zugrunde, einen Trägerkörper für Dosiereinrichtungen wie etwa Sprays, Zerstäubersysteme, Tropfflaschen oder gegebenenfalls auch Pinselflaschen, insbesondere zum Einsatz in Räumen, zur Aufnahme, Speicherung und geregelten Abgabe von Portionen flüchtiger Substanzen (Duft- bzw. Wirkstoffe) bereitzustellen, so daß einerseits eine unmittelbare Duftwirkung bei guter und gleichmäßiger Dosierbarkeit gewährleistet wird, dies andererseits jedoch über einen langen Zeitraum hinweg ermöglicht wird. Demzufolge zielt die vorliegende Erfindung auf einen Trägerkörper ab, der die nur bei Bedarf und in einer Portion zu applizierenden Duft- bzw. Wirkstoffe aufnimmt, ohne sie aufzusaugen. Die Wirkstoffkombination soll hierbei vom Trägerkörper in Abhängigkeit von der Temperatur mit konstant hoher Rate, kontrolliert und vollständig freigesetzt werden. In diesem Zusammenhang besteht eine weitere Aufgabe der Erfindung darin, einen Trägerkörper bereitzustellen, der diese kontinuierliche Verdunstung bzw. Sublimation der gespeicherten Substanzen in unveränderter Zusammensetzung garantiert.

Erfindungsgemäß wird diese Aufgabe durch einen Trägerkörper gemäß Anspruch 1 gelöst.

Somit ist eine kontinuierliche Verdunstung bzw. Sublimation der gespeicherten Substanzen in unveränderter Zusammensetzung über einen im Vergleich zu einer direkten Applikation einer Dosis in die Luft langen Zeitraum hinweg garantiert. Sowohl der Dufteindruck als auch der optische Eindruck des Trägerkörpers bleiben erhalten, ohne eine unerwünschte Veränderung zu erfahren. Durch die Anordnung mehrerer Vertiefungen in mindestens einer der Oberflächen des Trägerkörpers wird zudem eine sofortige Duftwirkung bei gleichzeitig kontinuierlicher Dosierbarkeit über einen im Vergleich zu einer direkten Applikation einer Dosis in die Luft langen Zeitraum hinweg gewährleistet.

Das Material des Trägerkörpers ist Metall oder eine Metallegierung mit einer Wärmeleitfähigkeit im Bereich von mindestens 10¹ W·m⁻¹·K⁻¹ bis höchstens 40·10¹ W·m⁻¹·K⁻¹, wobei es sich bei der Metallegierung vorzugsweise um Edelstahl mit einer Wärmeleitfähigkeit im Bereich von 1,5·10¹ W·m⁻¹·K⁻¹ handelt. Dementsprechend zieht man auf überraschend einfache und doch ausgesprochen wirkungsvolle Weise Nutzen aus der Tatsache, daß eine bestimmte Menge leicht flüchtiger Substanzen bei Raumtemperatur umso länger wahrgenommen werden kann, je höher die Wärmeleitfähigkeit des Materials ist, aus dem der Trägerkörper besteht. In bezug auf die vorliegende Erfindung bedeutet dies, daß die Wärmeleitfähigkeit des Trägermaterials umso höher sein sollte, je flüchtiger die Substanzen sind, um auf diese Weise eine Verlängerung der Duftwahrnehmung im Vergleich zu einer direkten Applikation in die Luft zu erreichen.

In einer besonders zweckmäßigen Weiterbildung ist der erfindungsgemäße Trägerkörper in Form mindestens einer Trägerplatte mit möglichst großer Oberfläche ausgebildet. In diesem Zusammenhang ist beispielsweise eine Ausgestaltungsform der vorliegenden Erfindung denkbar, in der der Trägerkörper in Form von zwei oder mehr Trägerplatten ausgebildet ist, die parallel zueinander angeordnet sind. Alternativ oder in Ergänzung hierzu kann der Trägerkörper auch in Form von zwei oder mehr Trägerplatten ausgebildet sein, die in lateraler Versetzung und/oder in angularer Versetzung zueinander angeordnet sind. In jedem der vorgenannten Fälle erweist es sich hierbei als zweckmäßig, wenn die zwei oder mehr Trägerplatten durch Verbindungsmittel miteinander verbunden sind.

Nach einer besonders vorteilhaften Ausführungsform der vorliegenden Erfindung kann die Trägerplatte in gekrümmter Form, insbesondere in Form einer Zylinderwand, eines Kegelmantels oder einer Kugelschale ausgebildet sein.

Bevorzugte Ausgestaltungen des erfindungsgemäßen Trägerkörpers beinhalten, daß die Trägerplatte eine Oberfläche im Bereich von mindestens 2 cm² bis höchstens 500 cm² und eine Dicke im Bereich von mindestens 0,1 mm bis höchstens 2 mm aufweist.

Nach einer besonders erfinderischen Weiterbildung des vorliegenden Trägerkörpers sind die Vertiefungen zumindest teilweise als Durchbrechungen ausgebildet, wobei das Verhältnis von Oberfläche zu Volumen des Trägerkörpers zweckmäßigerweise höchstens 500·10² m⁻¹ beträgt.

Nach einer weiteren vorteilhaften Ausführungsform der vorliegenden Erfindung sind die Vertiefungen bzw. Durchbrechungen in regelmäßigen Abständen angeordnet. Sind die Vertiefungen bzw. Durchbrechungen hierbei so ausgebildet, daß der Trägerkörper die Form einer Gaze, eines Gewebes, eines Gitters, eines Netzes oder eines Siebes aufweist, oder sind die Vertiefungen bzw. Durchbrechungen in Form von Rillen beliebiger Breite ausgebildet, so wird eine zusätzliche Verlängerung der Duftwahrnehmung erreicht.

Wenn der Trägerkörper in Form mindestens einer Trägerplatte mit möglichst großer Oberfläche ausgebildet ist, so ist in einer besonders bevorzugten Weiterbildung des erfindungsgemäßen Trägerkörpers die Tiefe der Vertiefungen bzw. Durchbrechungen in der Trägerplatte höchstens gleich der Dicke der Trägerplatte. Unabhängig davon bewegt sich der Durchmesser der Vertiefungen bzw. Durchbrechungen vorteilhafterweise im Bereich von mindestens 0,2 mm bis höchstens 1 mm, während sich der Mittelpunktsabstand benachbarter Vertiefungen bzw. Durchbrechungen im Bereich von mindestens 0,2 mm bis höchstens 60 mm bewegt.

Sind die im Trägerkörper angeordneten Vertiefungen bzw. Durchbrechungen nach Maßgabe vorstehend aufgeführter Dimensionierung höchstens vollständig mit den flüchtigen Substanzen (Duft- bzw. Wirkstoffen) angefüllt, so ist in hinreichendem Maße für die bei vorliegendem Einsatzgebiet des erfindungsgemäßen Trägerkörpers wünschenswerte Konvektion gesorgt, wobei die flüchtigen Substanzen in alle Richtungen verdampfbar sind.

Wie aus vorstehenden Ausführungen bereits zu entnehmen ist, ist für einen erfolgreichen Einsatz des erfindungsgemäßen Trägerkörpers seine physikalische Struktur von besonderer Bedeutung. Aus diesem Grund nehmen die Bereiche der Oberfläche der Trägerplatte, die Vertiefungen bzw. Durchbrechungen aufweisen, höchstens 60 % der gesamten Oberfläche der Trägerplatte ein.

Eine Verlängerung der Duftwahrnehmung wird auch durch ein zumindest abschnitts- oder stellenweises Beschichten des Trägerkörpers mit mindestens einem Medium erzielt, das in geeigneter Weise die Haftung der flüchtigen Substanzen erhöht. Hierbei ist das Medium vorzugsweise licht- und/oder oxidationsbeständig sowie vorteilhafterweise geruchsneutral. Bei dem Medium kann es sich beispielsweise um einen Fixateur, der beispielsweise durch einen Zerstäuber auf den Trägerkörper aufgetragen ist, oder um Öl handeln.

Beim praktischen Einsatz hat der Trägerkörper beim Applizieren der flüchtigen Substanzen von einer Dosiereinrichtung auf den Trägerkörper zweckmäßigerweise einen Abstand von höchstens 20 cm von der Dosiereinrichtung.

Die Erfindung wird nachfolgend anhand der in den Fig. 1 bis 13 schematisch dargestellten, teilweise stark vergrößert abgebildeten Ausführungsbeispiele näher erläutert. Es zeigen:
- Fig. 1: eine schematische Aufsicht auf eine erste Ausführungsform eines erfindungsgemäßen Trägerkörpers zur geregelten Abgabe flüchtiger Substanzen;
- Fig. 2: eine schematische Aufsicht auf eine zweite Ausführungsform eines erfindungsgemäßen Trägerkörpers zur geregelten Abgabe flüchtiger Substanzen;
- Fig. 3: eine schematische Aufsicht auf eine dritte Ausführungsform eines erfindungsgemäßen Trägerkörpers zur geregelten Abgabe flüchtiger Substanzen;
- Fig. 4: einen vergrößerten Ausschnitt der in Fig. 3 dargestellten schematischen Aufsicht auf die dritte Ausführungsform eines erfindungsgemäßen Trägerkörpers;
- Fig. 5: eine seitliche Schnittansicht des in Fig. 4 dargestellten vergrößerten Ausschnitts der schematischen Aufsicht auf die dritte Ausführungsform eines erfindungsgemäßen Trägerkörpers entlang der Schnittlinie S4-S4 aus Fig. 4;
- Fig. 6: einen vergrößerten Ausschnitt einer schematischen Aufsicht auf eine vierte Ausführungsform eines erfindungsgemäßen Trägerkörpers zur geregelten Abgabe flüchtiger Substanzen;
- Fig. 7: einen vergrößerten Ausschnitt einer schematischen Aufsicht auf eine fünfte Ausführungsform eines erfindungsgemäßen Trägerkörpers zur geregelten Abgabe flüchtiger Substanzen;
- Fig. 8: einen vergrößerten Ausschnitt einer schematischen Aufsicht auf eine sechste Ausführungsform eines erfindungsgemäßen Trägerkörpers zur geregelten Abgabe flüchtiger Substanzen;
- Fig. 9: einen vergrößerten Ausschnitt einer schematischen Aufsicht auf eine siebte Ausführungsform eines erfindungsgemäßen Trägerkörpers zur geregelten Abgabe flüchtiger Substanzen;
- Fig. 10: eine schematische Ansicht einer achten Ausführungsform eines erfindungsgemäßen Trägerkörpers zur geregelten Abgabe flüchtiger Substanzen;
- Fig. 11: eine schematische Ansicht einer neunten Ausführungsform eines erfindungsgemäßen Trägerkörpers zur geregelten Abgabe flüchtiger Substanzen;
- Fig. 12: eine schematische Aufsicht auf eine zehnte Ausführungsform eines erfindungsgemäßen Trägerkörpers zur geregelten Abgabe flüchtiger Substanzen; und
- Fig. 13: einen Raumlufterfrischer mit einer elften Ausführungsform eines erfindungsgemäßen Trägerkörpers zur geregelten Abgabe flüchtiger Substanzen.

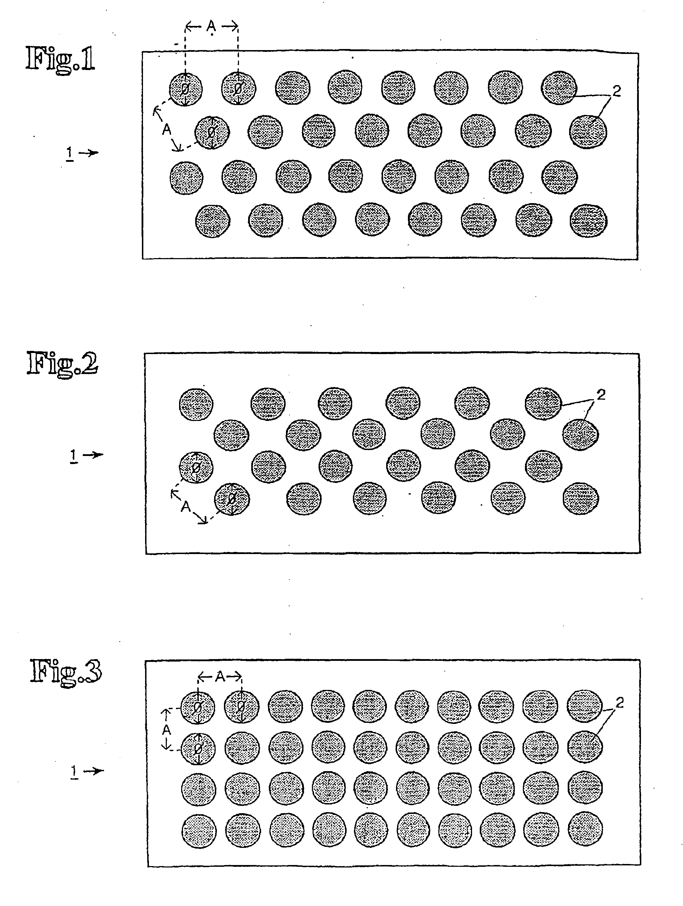

Die Fig. 1 bis 12 stellen schematische Ansichten von zehn Ausführungsformen eines erfindungsgemäßen Trägerkörpers zur Aufnahme, Speicherung und geregelten Abgabe flüchtiger Substanzen (Duft- bzw. Wirkstoffe) dar, die dosierbar auf den Trägerkörper aufbringbar sind. Der Trägerkörper ermöglicht hierbei erfindungsgemäß über einen langen Zeitraum hinweg eine unmittelbare Duftwirkung bei gleichzeitiger Dosierbarkeit, was eine kontinuierliche Verdunstung bzw. Sublimation der gespeicherten Substanzen in unveränderter Zusammensetzung über einen im Vergleich zu einer direkten Applikation einer Dosis in die Luft langen Zeitraum hinweg garantiert. Der Dufteindruck bleibt somit für eine große Zeitspanne auf dem Trägerkörper erhalten, ohne eine unerwünschte Veränderung zu erfahren. Anzumerken ist an dieser Stelle noch, daß eine zusätzliche Verlängerung der Duftwahrnehmung auch durch ein zumindest abschnitts- oder stellenweises Beschichten des Trägerkörpers mit mindestens einem Medium erzielt wird, das die Haftung der flüchtigen Substanzen erhöht. Hierbei ist das Medium licht- und oxidationsbeständig sowie geruchsneutral. Bei dem Medium kann es sich beispielsweise um einen Fixateur, der beispielsweise durch einen Zerstäuber auf den Trägerkörper aufgetragen ist, oder um Öl handeln. Unter Fixateuren versteht man in diesem Zusammenhang Stoffe, die imstande sind, dem Duft der flüchtigen Substanzen eine erhöhte Beständigkeit zu verleihen und die Verdunstung der einzelnen Duftkomponenten so zu verlangsamen und einander anzugleichen, daß der Duftcharakter während der Verdunstungszeit einigermaßen konstant bleibt. Die Fixateure selbst sind hierbei meist schwerflüchtig sowie hochsiedend und können duftend oder duftfrei sein.

Gemeinsam ist den in den Fig. 1 bis 12 schematisch dargestellten Ausführungsformen, daß jeder der dargestellten Trägerkörper Vertiefungen bzw. Durchbrechungen 2 zur Aufnahme und Speicherung der flüchtigen Substanzen aufweist. Wie vorstehend bereits ausgeführt, ist für einen erfolgreichen Einsatz des erfindungsgemäßen Trägerkörpers seine physikalische Struktur von besonderer Bedeutung. Aus diesem Grund sind die in den Fig. 1 bis 12 gezeigten erfindungsgemäßen Trägerkörper flächig ausgebildet, so daß das Verhältnis von Oberfläche zu Volumen bei diesen Trägerkörpern mindestens 1·10² m⁻¹ beträgt. Abhängig von der konkreten Ausgestaltung und Anzahl der Vertiefungen bzw. Durchbrechungen 2 zur Aufnahme und Speicherung der flüchtigen Substanzen liegt der obere Grenzwert für das Verhältnis von Oberfläche zu Volumen bei diesen Trägerkörpern bei 500·10² m⁻¹. Hierbei bewegt sich das Verhältnis von Oberfläche zu Volumen vorzugsweise in einem Bereich von 5·10² m⁻¹ bis 100·10² m⁻¹.

Des weiteren nehmen die Bereiche der Oberfläche(n) des Trägerkörpers, die Vertiefungen bzw. Durchbrechungen 2 aufweisen, in den Fig. 1 bis 12 höchstens 60 % der gesamten Oberfläche des Trägerkörpers ein. Der Trägerkörper weist hierbei eine Oberfläche im Bereich von mindestens 2 cm² bis höchstens 500 cm² und eine Dicke (D) im Bereich von mindestens 0,1 mm bis höchstens 2 mm auf.

Nicht explizit den Fig. 1 bis 12 entnehmbar ist hingegen, daß die erfindungsgemäßen Trägerkörper aus einem Material bestehen, das gegen die flüchtigen Substanzen und/oder deren Lösungsmittel chemisch inert ist. Dieses Material umfasst Metall oder eine Metallegierung mit einer Wärmeleitfähigkeit im Bereich von mindestens 10¹ W·m⁻¹·K⁻¹ bis höchstens 40·10¹ W·m⁻¹·K⁻¹, wobei es sich bei der Metallegierung um Edelstahl mit einer Wärmeleitfähigkeit im Bereich von 1,5·10¹ W·m⁻¹·K⁻¹ handeln kann. Da nun eine bestimmte Menge leicht flüchtiger Substanzen bei Raumtemperatur umso länger wahrgenommen wird, je höher die Wärmeleitfähigkeit des Materials ist, aus dem der Trägerkörper besteht, bedeutet dies in bezug auf die dargestellten Ausführungsformen, daß die Wärmeleitfähigkeit des Trägermaterials umso höher sein sollte, je flüchtiger die Substanzen sind, um auf diese Weise eine Verlängerung der Duftwahrnehmung im Vergleich zu einer direkten Applikation in die Luft zu erreichen. Dies impliziert andererseits, daß bei einer niedrigen Wärmeleitfähigkeit des Trägermaterials zusätzliche Maßnahmen zur Verlängerung der Duftwahrnehmung ergriffen werden können. Die Substanzen sollten hierbei möglichst großflächig auf den Trägerkörper appliziert, das heißt zerstäubt, gesprüht oder gepinselt werden.

In den Fig. 1, 2 und 3 ist der erfindungsgemäße Trägerkörper jeweils in Form einer Trägerplatte 1 mit möglichst großer äußerer Oberfläche ausgebildet. Hierbei sind die Vertiefungen bzw. Durchbrechungen 2 in der Trägerplatte 1 jeweils in regelmäßigen Abständen in Form kreisrunder Löcher ausgestaltet, deren Durchmesser Ø sich im Bereich von mindestens 0,2 mm bis höchstens 1 mm bewegt, während sich ihr Mittelpunktsabstand A im Bereich von mindestens 0,2 mm bis höchstens 60 mm bewegt.

Im speziellen errechnet sich bei der in Fig. 1 gezeigten ersten Ausführungsform (RV = Rundlochung versetzt) eines erfindungsgemäßen Trägerkörpers zur geregelten Abgabe flüchtiger Substanzen der prozentuale Anteil Aₒ der offenen Fläche an der gesamten Oberfläche zu Aₒ = 90,7·(Ø/A)², wohingegen bei den in Fig. 2 bzw. Fig. 3 gezeigten zweiten bzw. dritten Ausführungsformen (RD = Rundlochung diagonal bzw. RG = Rundlochung gerade) eines erfindungsgemäßen Trägerkörpers zur geregelten Abgabe flüchtiger Substanzen der prozentuale Anteil Aₒ der offenen Fläche an der gesamten Oberfläche jeweils Aₒ = 78,5·(∅/A)² beträgt.

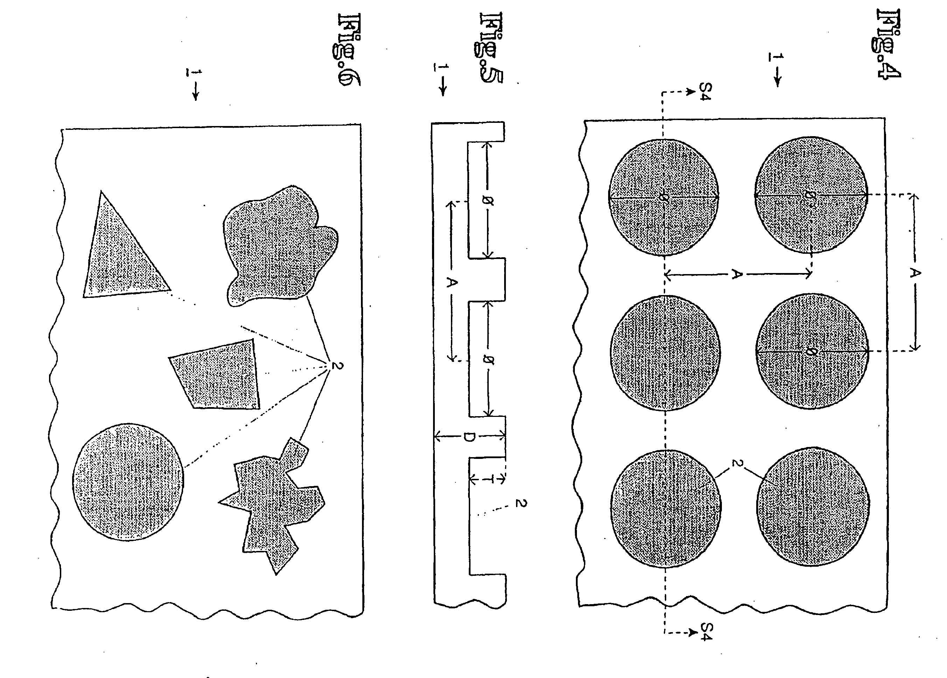

Zur besseren Veranschaulichung vorstehender Kenngrößen stellt Fig. 4 einen vergrößerten Ausschnitt der in Fig. 3 dargestellten schematischen Aufsicht auf die dritte Ausführungsform eines erfindungsgemäßen Trägerkörpers dar. Fig. 5 zeigt eine seitliche Schnittansicht des in Fig. 4 dargestellten vergrößerten Ausschnitts der schematischen Aufsicht auf die dritte Ausführungsform eines erfindungsgemäßen Trägerkörpers entlang der Schnittlinie S4-S4 aus Fig. 4, wobei in Fig. 5 die Tiefe T der Vertiefungen bzw. Durchbrechungen 2 gleich der halben Dicke D der Trägerplatte 1 ist.

Verwendet man bei den in den Fig. 1, 2 und 3 jeweils gewählten Dimensionierungen als Trägermaterial beispielsweise Polyethylen (PE) mit einer Wärmeleitfähigkeit im Bereich von 1,2·10⁻¹ W·m⁻¹·K⁻¹, nicht erfindungsgemäß so wird durch die gezeigte Anordnung der Löcher eine Verlängerung der Duftwahrnehmung von einer halben Stunde auf neun Stunden, das heißt um den Faktor 18 beobachtet.

Anzumerken ist in diesem Zusammenhang noch, daß der Durchmesser ∅ der in der Trägerplatte 1 angeordneten Löcher auch von der Art der Applikation der flüchtigen Substanz abhängig ist: Werden die flüchtigen Substanzen beispielsweise aus einer Spraydose auf die Trägerplatte 1 appliziert, sollten die Löcher einen Durchmesser ∅ in der Größenordnung von etwa 0,2 mm aufweisen, während für die Applikation mit mechanischen Zerstäubern Lochdurchmesser ∅ im Bereich von etwa 0,6 mm überzeugende Ergebnisse hervorrufen. In jedem Falle ist die obere Grenze für den Lochdurchmesser ∅ durch den Anteil durchtretender Tröpfchen gegeben. Es sollten zwar möglichst viele Tröpfchen auf der Trägerplatte 1 haften, jedoch keinen geschlossenen Film bilden. Hierbei sollten die in der Trägerplatte 1 angeordneten Löcher nach Maßgabe vorstehend aufgeführter Dimensionierung nicht vollständig mit den flüchtigen Substanzen angefüllt sein, so daß in hinreichendem Maße für die bei vorliegendem Einsatzgebiet des erfindungsgemäßen Trägerkörpers wünschenswerte Konvektion gesorgt ist.

Fig. 6 zeigt eine schematische Aufsicht auf eine vierte Ausführungsform eines erfindungsgemäßen Trägerkörpers zur geregelten Abgabe flüchtiger Substanzen, bei der unregelmäßig geformte Vertiefungen bzw. Durchbrechungen 2 in der Trägerplatte 1 in unregelmäßigen Abständen angeordnet sind.

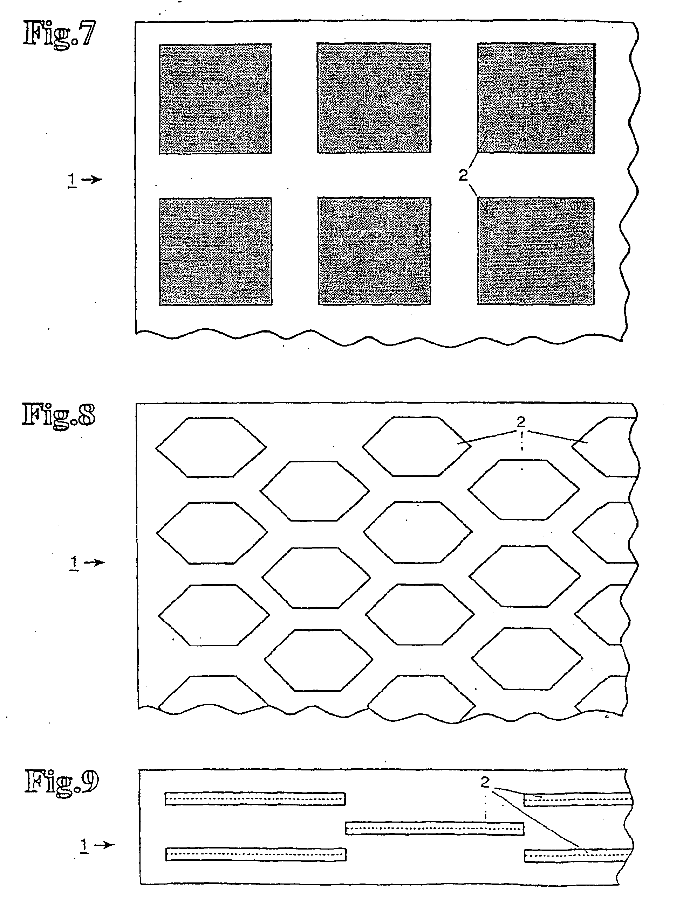

Es wurde vorstehend bereits mehrfach ausgeführt, daß die Vertiefungen bzw. Durchbrechungen 2 in der Trägerplatte 1 auch so ausgebildet sein können, daß die Trägerplatte 1 die Form einer Gaze, eines Gewebes, eines Gitters, eines Netzes oder eines Siebes aufweist. So sind in der fünften und sechsten Ausführungsform eines erfindungsgemäßen Trägerkörpers zur geregelten Abgabe flüchtiger Substanzen, die in den Fig. 7 und 8 in schematischer Aufsicht dargestellt sind, die Vertiefungen bzw. Durchbrechungen 2 so ausgebildet, daß die Trägerplatte 1 die Form eines Gitters mit quadratischer Struktur (Fig. 7) bzw. mit Wabenstruktur (Fig. 8) aufweist.

Fig. 9 zeigt eine schematische Aufsicht auf eine siebte Ausführungsform eines erfindungsgemäßen Trägerkörpers zur geregelten Abgabe flüchtiger Substanzen, bei der die Vertiefungen bzw. Durchbrechungen 2 in Form von Rillen ausgebildet sind, durch die, wie durch die quadratischen Vertiefungen der Fig. 7 und die wabenförmigen Durchbrechungen der Fig. 8, eine zusätzliche Verlängerung der Duftwahrnehmung erreicht wird.

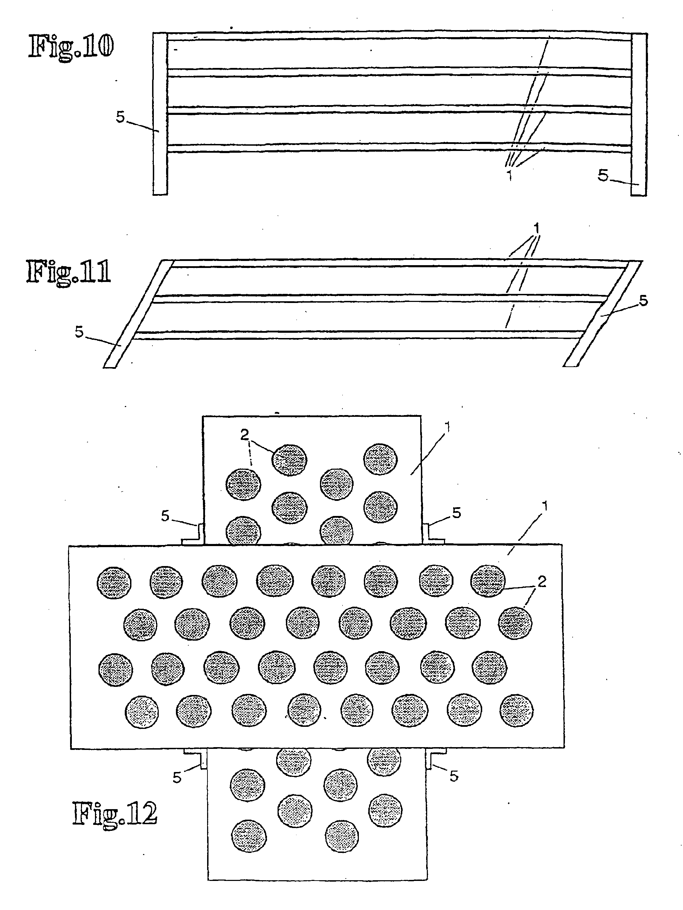

Fig. 10 zeigt eine schematische Ansicht einer achten Ausführungsform eines erfindungsgemäßen Trägerkörpers zur geregelten Abgabe flüchtiger Substanzen. Dieser Trägerkörper ist in Form von vier Trägerplatten 1 ausgebildet, die parallel zueinander angeordnet sind und durch Verbindungsmittel 5 nicht nur miteinander verbunden, sondern auch in dieser Position stabilisiert und durch die Verbindungsmittel 5 getragen werden.

Durch die Anordnung von mehr als einer Trägerplatte 1 wird hierbei weder die erfindungswesentliche kontinuierliche Verdunstung bzw. Sublimation der gespeicherten Substanzen in unveränderter Zusammensetzung über einen im Vergleich zu einer direkten Applikation einer Dosis in die Luft langen Zeitraum hinweg noch der Dufteindruck und/oder der optische Eindruck des Trägerkörpers in irgendeiner Form beeinträchtigt; vielmehr werden die vorgenannten Effekte in erwünschter Weise verstärkt und über einen längeren Zeitraum hinweg aufrechterhalten.

Das gleiche gilt auch für die in Fig. 11 gezeigte schematische Ansicht einer neunten Ausführungsform eines erfindungsgemäßen Trägerkörpers zur geregelten Abgabe flüchtiger Substanzen. Dieser Trägerkörper ist in Form von drei Trägerplatten 1 ausgebildet, die in lateraler Versetzung zueinander angeordnet sind und durch Verbindungsmittel 5 nicht nur miteinander verbunden, sondern auch in dieser Position stabilisiert und durch die Verbindungsmittel 5 getragen werden.

Fig. 12 zeigt eine schematische Aufsicht auf eine zehnte Ausführungsform eines erfindungsgemäßen Trägerkörpers zur geregelten Abgabe flüchtiger Substanzen. Dieser Trägerkörper ist in Form von zwei der in Fig. 1 dargelegten Trägerplatten 1 ausgebildet, die in angularer Versetzung, im gezeigten Beispiel der Fig. 12 in rechtwinklig angularer Versetzung, zueinander angeordnet sind und durch winkelförmige Verbindungsmittel 5 nicht nur miteinander verbunden, sondern auch in dieser Position stabilisiert und durch die Verbindungsmittel 5 getragen werden. Es versteht sich hierbei von selbst, daß die angulare Versetzung auch jeden von 90° verschiedenen Wert annehmen kann.

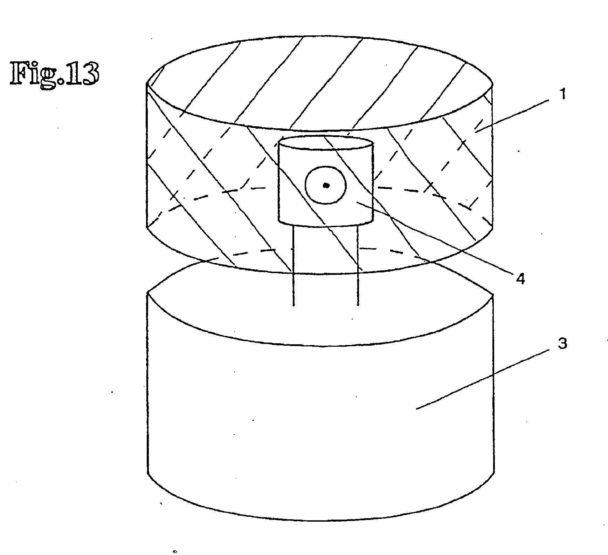

Fig. 13 zeigt einen Raumlufterfrischer mit einer elften Ausführungsform eines erfindungsgemäßen Trägerkörpers zur geregelten Abgabe flüchtiger Substanzen. Ein im Prinzip beliebig gestaltbares Gefäß 3 ist mit flüssigen Duft- bzw. Wirkstoffen gefüllt, wobei die Dosierung der flüssigen Duft- bzw. Wirkstoffe durch eine Dosiereinrichtung 4 wie etwa einen Zerstäuber erfolgt. In Fig. 13 ist die erfindungsgemäße Trägerplatte 1, die zur Aufnahme und Verdunstung der Wirkstoffportion bestimmt ist, in gekrümmter Form ausgebildet; sie besteht aus Edelstahl, ist durchbrochen und ist in Form einer Zylinderwand konzentrisch um die Dosiereinrichtung 4 herum angebracht, wobei die Trägerplatte 1 beim Applizieren der flüchtigen Substanzen einen Abstand von höchstens 20 cm von der Dosiereinrichtung 4 hat. Anzumerken ist an dieser Stelle zu Fig. 13 noch, daß die Trägerplatte 1 selbstverständlich auch in Form beispielsweise eines Kegelmantels oder einer Kugelschale ausgebildet sein könnte.

### Beispiel:

Abschließend sei noch ein beispielhaftes Experiment aufgeführt, bei dem 0,1 ml eines Eau de Toilette durch Sprühen mittels eines mechanischen Zerstäubers auf eine geeignete Auswahl der vorstehend genannten Trägermaterialien bzw. (als Referenz) in die Luft appliziert wurde. Die Duftdauer wurde in 50 m³ großen Räumen durch verschiedene Personen ermittelt, wobei die Versuche bei einer Raumtemperatur von 20 °C ± 1 °C durchgeführt wurden. In nachfolgender Tabelle sind in der linken Spalte die verschiedenen Trägermaterialien aufgeführt; der rechten Spalte ist die Duftdauer (gemessen in Stunden) zu entnehmen, jeweils bei einer Trägerplatte 1 im undurchbrochenen Zustand und im durchbrochenen Zustand (siehe mittlere Spalte):

| *Trägermaterial* | *Trägerplatte durchbrochen* | *Duftdauer in Stunden* |
|---|---|---|
| Polyethylen (PE) | nein | 3 |
| | ja | 20 |
| | | |
| Stahl 1.4301 | nein | 7 |
| | ja | 33 |
| | | |
| Luft | | 2,5 |

## Patentansprüche

1. Trägerkörper zur geregelten Abgabe flüchtiger Substanzen, die dosierbar auf den Trägerkörper aufbringbar sind, wobei der Trägerkörper flächig ausgebildet ist, mindestens eine der Oberflächen des Trägerkörpers mehrere Vertiefungen (2) zur Aufnahme und Speicherung der flüchtigen Substanzen aufweist, das Verhältnis von Oberfläche zu Volumen des Trägerkörpers mindestens 1·10² m-1 beträgt, der Trägerkörper aus einem Material besteht, das gegen die flüchtigen Substanzen und/oder deren Lösungsmittel chemisch inert ist und das Material des Trägerkörpers Metall oder eine Metallegierung mit einer Wärmeleitfähigkeit im Bereich von mindestens 10¹ W·m⁻¹·K⁻¹ bis höchstens 40·10¹ W·m⁻¹·K⁻¹ ist.

2. Trägerkörper nach Anspruch 1, **dadurch gekennzeichnet, daß** es sich bei der Metallegierung um Edelstahl mit einer Wärmeleitfähigkeit im Bereich von 1,5·10¹ W·m⁻¹·K⁻¹ handelt.

3. Trägerkörper nach Anspruch 1 oder 2, **dadurch gekennzeichnet, daß** der Trägerkörper in Form mindestens einer Trägerplatte (1) mit möglichst großer Oberfläche ausgebildet ist.

4. Trägerkörper nach Anspruch 3, **dadurch gekennzeichnet, daß** der Trägerkörper in Form von zwei oder mehr Trägerplatten (1) ausgebildet ist, die parallel zueinander angeordnet sind.

5. Trägerkörper nach Anspruch 3 oder 4, **dadurch gekennzeichnet, daß** der Trägerkörper in Form von zwei oder mehr Trägerplatten (1) ausgebildet ist, die in lateraler Versetzung zueinander angeordnet sind.

6. Trägerkörper nach einem der Ansprüche 3 bis 5, **dadurch gekennzeichnet, daß** der Trägerkörper in Form von zwei oder mehr Trägerplatten (1) ausgebildet ist, die in angularer Versetzung zueinander angeordnet sind.

7. Trägerkörper nach einem der Ansprüche 4 bis 6, **dadurch gekennzeichnet, daß** die zwei oder mehr Trägerplatten (1) durch Verbindungsmittel (5) miteinander verbunden sind.

8. Trägerkörper nach einem der Ansprüche 3 bis 7, **dadurch gekennzeichnet, daß** die Trägerplatte (1) in gekrümmter Form ausgebildet ist.

9. Trägerkörper nach Anspruch 8, **dadurch gekennzeichnet, daß** die Trägerplatte (1) in Form einer Zylinderwand, eines Kegelmantels oder einer Kugelschale ausgebildet ist.

10. Trägerkörper nach einem der Ansprüche 3 bis 9, **dadurch gekennzeichnet, daß** die Trägerplatte (1) eine Oberfläche im Bereich von mindestens 2 cm² bis höchstens 500 cm² aufweist.

11. Trägerkörper nach einem der Ansprüche 3 bis 10, **dadurch gekennzeichnet, daß** die Trägerplatte (1) eine Dicke (D) im Bereich von mindestens 0,1 mm bis höchstens 2 mm aufweist.

12. Trägerkörper nach einem der Ansprüche 1 bis 11, **dadurch gekennzeichnet, daß** die Vertiefungen (2) zumindest teilweise als Durchbrechungen (2) ausgebildet sind.

13. Trägerkörper nach Anspruch 12, **dadurch gekennzeichnet, daß** das Verhältnis von Oberfläche zu Volumen des Trägerkörpers höchstens 500·10² m⁻¹ beträgt.

14. Trägerkörper nach Anspruch 12 oder 13, **dadurch gekennzeichnet, daß** die Vertiefungen bzw. Durchbrechungen (2) in regelmäßigen Abständen angeordnet sind.

15. Trägerkörper nach einem der Ansprüche 12 bis 14, **dadurch gekennzeichnet, daß** die Vertiefungen bzw. Durchbrechungen (2) so ausgebildet sind, daß der Trägerkörper die Form einer Gaze, eines Gewebes, eines Gitters, eines Netzes oder eines Siebes aufweist.

16. Trägerkörper nach einem der Ansprüche 12 bis 15, **dadurch gekennzeichnet, daß** die Vertiefungen bzw. Durchbrechungen (2) in Form von Rillen beliebiger Breite ausgebildet sind.

17. Trägerkörper nach Anspruch 11 und nach einem der Ansprüche 15 bis 19, **dadurch gekennzeichnet, daß** die Tiefe (T) der Vertiefungen bzw. Durchbrechungen (2) höchstens gleich der Dicke (D) der Trägerplatte (1) ist.

18. Trägerkörper nach einem der Ansprüche 12 bis 17, **dadurch gekennzeichnet, daß** sich der Durchmesser (Ø) der Vertiefungen bzw. Duschbrechungen (2) im Bereich von mindestens 0,2 mm bis höchstens 1 mm bewegt.

19. Trägerkörper nach einem der Ansprüche 12 bis 18, **dadurch gekennzeichnet, daß** sich der Mittelpunktsabstand (A) benachbarter Vertiefungen bzw. Durchbrechungen (2) im Bereich von mindestens 0,2 mm bis höchstens 60 mm bewegt.

20. Trägerkörper nach einem der Ansprüche 12 bis 19, **dadurch gekennzeichnet, daß** die Bereiche der Oberfläche(n) des Trägerkörpers, die Vertiefungen bzw. Durchbrechungen (2) aufweisen, höchstens 60 % der gesamten Oberfläche des Trägerkörpers einnehmen.

21. Trägerkörper nach einem der Ansprüche 1 bis 20, **dadurch gekennzeichnet, daß** der Trägerkörper zumindest abschnitts- oder stellenweise mit mindestens einem Medium beschichtet ist.

22. Trägerkörper nach Anspruch 21, **dadurch gekennzeichnet, daß** das Medium die Haftung der flüchtigen Substanzen erhöht.

23. Trägerkörper nach Anspruch 21 oder 22, **dadurch gekennzeichnet, daß** das Medium licht- und/oder oxidationsbeständig ist.

24. Trägerkörper nach einem der Ansprüche 21 bis 23, **dadurch gekennzeichnet, daß** das Medium geruchsneutral ist.

25. Trägerkörper nach einem der Ansprüche 21 bis 24, **dadurch gekennzeichnet, daß** es sich bei dem Medium um einen Fixateur handelt.

26. Trägerkörper nach einem der Ansprüche 21 bis 25, **dadurch gekennzeichnet, daß** es sich bei dem Medium um Öl handelt.

27. Trägerkörper nach einem der Ansprüche 1 bis 26, **dadurch gekennzeichnet, daß** der Trägerkörper beim Applizieren der flüchtigen Substanzen von einer Dosiereinrichtung auf den Trägerkörper einen Abstand von höchstens 20 cm von der Dosiereinrichtung hat.

## Claims

1. Support body for the controlled release of volatile substances which may be applied to the support body in a metered manner, wherein the support body is of a flat construction, at least one of the surfaces of the support body has a plurality of recesses (2) for receiving and holding the volatile substances, the ratio of surface area to volume of the support body is at least 1 × 10² m⁻¹, the support body consists of a material which is chemically inert to the volatile substances and/or their solvents and the material of the support body is metal or a metal alloy having a thermal conductivity in the range from a minimum of 10¹ W·m⁻¹·K⁻¹ to a maximum of 40·10¹ W·m⁻¹·K⁻¹.

2. Support body according to Claim 1, **characterized in that** the metal alloy is stainless steel having a thermal conductivity in the region of 1.5·10¹ W·m⁻¹ ·K⁻¹.

3. Support body according to Claim 1 or 2, **characterized in that** the support body is constructed in the form of at least one carrier plate (1) having as large as possible a surface area.

4. Support body according to Claim 3, **characterized in that** the support body is constructed in the form of two or more carrier plates (1) which are disposed in parallel to one another.

5. Support body according to Claim 3 or 4, **characterized in that** the support body is constructed in the form of two or more carrier plates (1) which are disposed in lateral displacement to one another.

6. Support body according to one of Claims 3 to 5, **characterized in that** the support body is constructed in the form of two or more carrier plates (1) which are disposed in angular displacement to one another.

7. Support body according to one of Claims 4 to 6, **characterized in that** the two or more carrier plates (1) are connected to one another by connection means (5).

8. Support body according to one of Claims 3 to 7, **characterized in that** the support plate (1) is constructed in curved shape.

9. Support body according to Claim 8, **characterized in that** the support plate (1) is constructed in the form of a cylindrical wall, a conical surface or a spherical shell.

10. Support body according to one of Claims 3 to 9, **characterized in that** the carrier plate (1) has a surface area in the range from a minimum of 2 cm² to a maximum of 500 cm².

11. Support body according to one of Claims 3 to 10, **characterized in that** the carrier plate (1) has a thickness (D) in the range from a minimum of 0.1 mm to a maximum of 2 mm.

12. Support body according to one of Claims 1 to 11, **characterized in that** the recesses (2) are constructed at least in part as breakthroughs (2).

13. Support body according to Claim 12, **characterized in that** the ratio of surface area to volume of the support body is a maximum of 500·10² m⁻¹.

14. Support body according to Claim 12 or 13, **characterized in that** the recesses or breakthroughs (2) are disposed at regular intervals.

15. Support body according to one of Claims 12 to 14, **characterized in that** the recesses or breakthroughs (2) are constructed in such a manner that the support body has the form of a gauze, a fabric, a grating, a net or a screen.

16. Support body according to one of Claims 12 to 15, **characterized in that** the recesses or breakthroughs (2) are constructed in the form of grooves of any width.

17. Support body according to Claim 11 and according to one of Claims 15 to 19, **characterized in that** the depth (T) of the recesses or breakthroughs (2) is at most equal to the thickness (D) of the carrier plate (1).

18. Support body according to one of Claims 12 to 17, **characterized in that** the diameter (Ø) of the recesses or breakthroughs (2) ranges from a minimum of 0.2 mm to a maximum of 1 mm.

19. Support body according to one of Claims 12 to 18, **characterized in that** the distance between centres (A) of adjacent recesses or breakthroughs (2) ranges from a minimum of 0.2 mm to a maximum of 60 mm.

20. Support body according to one of Claims 12 to 19, **characterized in that** the regions of the surface(s) of the support body which have recesses or breakthroughs (2) occupy a maximum of 60% of the total surface area of the support body.

21. Support body according to one of Claims 1 to 20, **characterized in that** the support body is coated at least in sections or points with at least one medium.

22. Support body according to Claim 21, **characterized in that** the medium increases the adhesion of the volatile substances.

23. Support body according to Claim 21 or 22, **characterized in that** the medium is light-resistant and/or oxidation-resistant.

24. Support body according to one of Claims 21 to 23, **characterized in that** the medium is odour-neutral.

25. Support body according to one of Claims 21 to 24, **characterized in that** the medium is a fixative.

26. Support body according to one of Claims 21 to 25, **characterized in that** the medium is oil.

27. Support body according to one of Claims 1 to 26, **characterized in that** the support body, when the volatile substances are applied from a metering device onto the support body, has a distance of a maximum of 20 cm from the metering device.

## Revendications

1. Substrat destiné à la libération régulée de substances volatiles dont l'application sur le substrat peut être dosée, dans lequel le substrat a une conformation plane, au moins l'une de ses faces présente plusieurs cavités (2) destinées à recevoir et à stocker les substances volatiles, le rapport de la surface au volume du substrat s'élevant au moins à 1.10² m⁻¹, le substrat est réalisé en une matière qui est chimiquement inerte vis-à-vis des substances volatiles et/ou de leurs solvants, et la matière qui le constitue est un métal ou un alliage métallique ayant une conductivité thermique allant d'un minimum de 10¹ W.m⁻¹.K⁻¹ à un maximum de 40.10¹ W.m⁻¹.K⁻¹.

2. Substrat suivant la revendication 1, **caractérisé en ce que** l'alliage métallique consiste en un acier spécial ayant une conductivité thermique de l'ordre de 1,5.10¹ W.m⁻¹.K⁻¹.

3. Substrat suivant la revendication 1 ou 2, **caractérisé en ce qu'**il est réalisé sous forme d'au moins une plaque de support (1) de surface la plus grande possible.

4. Substrat suivant la revendication 3, **caractérisé en ce qu'**il est réalisé sous forme de deux ou plus de deux plaques de support (1) qui sont disposées en parallélisme.

5. Substrat suivant la revendication 3 ou 4, **caractérisé en ce qu'**il est réalisé sous forme de deux ou plus de deux plaques de support (1) qui présentent entre elles un décalage latéral.

6. Substrat suivant l'une des revendications 3 à 5, **caractérisé en ce qu'**il est réalisé sous forme de deux ou plus de deux plaques de support (1) qui présentent entre elles un décalage angulaire.

7. Substrat suivant l'une des revendications 4 à 6, **caractérisé en ce que** les plaques de support (1) au nombre de deux au moins sont liées entre elles par des éléments de liaison (5).

8. Substrat suivant l'une des revendications 3 à 7, **caractérisé en ce que** la plaque de support (1) est réalisée avec une forme courbe.

9. Substrat suivant la revendication 8, **caractérisé en ce que** la plaque de support (1) est réalisée sous forme d'une paroi cylindrique, d'une enveloppe conique ou d'une coque sphérique.

10. Substrat suivant l'une des revendications 3 à 9, **caractérisé en ce que** la plaque de support (1) présente une surface allant d'un minimum de 2 cm² à un maximum de 500 cm².

11. Substrat suivant l'une des revendications 3 à 10, **caractérisé en ce que** la plaque de support (1) présente une épaisseur (D) allant d'un minimum de 0,1 mm à un maximum de 2 mm.

12. Substrat suivant l'une des revendications 1 à 11, **caractérisé en ce que** les cavités (2) sont réalisées au moins partiellement comme des découpures (2).

13. Substrat suivant la revendication 12, **caractérisé en ce que** le rapport de sa surface à son volume s'élève au maximum à 500.10² m⁻¹.

14. Substrat suivant la revendication 12 ou 13, **caractérisé en ce que** les cavités ou les découpures (2) sont disposées à des intervalles réguliers.

15. Substrat suivant l'une des revendications 12 à 14, **caractérisé en ce que** les cavités ou les découpures (2) sont réalisées de manière que le substrat présente la forme d'une gaze, d'un tissu, d'une grille, d'un filet ou d'un tamis.

16. Substrat suivant l'une des revendications 12 à 15, **caractérisé en ce que** les cavités ou les découpures (2) sont réalisées sous forme de rainures de largeur quelconque.

17. Substrat suivant la revendication 11 et suivant l'une des revendications 15 à 19, **caractérisé en ce que** la profondeur (T) des cavités ou des découpures (2) est au maximum égale à l'épaisseur (D) de la plaque de support (1).

18. Substrat suivant l'une des revendications 12 à 17, **caractérisé en ce que** le diamètre (ø) des cavités ou des découpures (2) varie dans la plage allant d'un minimum de 0,2 mm à un maximum de 1 mm.

19. Substrat suivant l'une des revendications 12 à 18, **caractérisé en ce que** la distance entre centres (A) de cavités ou de découpures (2) voisines varie dans une plage allant d'un minimum de 0,2 mm à un maximum de 60 mm.

20. Substrat suivant l'une des revendications 12 à 19, **caractérisé en ce que** les zones de sa surface ou de ses surfaces qui présentent des cavités ou des découpures (2) occupent au maximum 60 % de la surface totale du substrat.

21. Substrat suivant l'une des revendications 1 à 20, **caractérisé en ce qu'**il est revêtu d'au moins un milieu par secteurs ou par places.

22. Substrat suivant la revendication 21, **caractérisé en ce que** le milieu accroît la fixation des substances volatiles.

23. Substrat suivant la revendication 21 ou 22, **caractérisé en ce que** le milieu est stable à la lumière et/ou à l'oxydation.

24. Substrat suivant l'une des revendications 21 à 23, **caractérisé en ce que** le milieu est neutre du point de vue olfactif.

25. Substrat suivant l'une des revendications 21 à 24, **caractérisé en ce que** le milieu est un fixateur.

26. Substrat suivant l'une des revendications 21 à 25, **caractérisé en ce que** le milieu est une huile.

27. Substrat suivant l'une des revendications 1 à 26, **caractérisé en ce qu'**au moment de l'application des substances volatiles par un dispositif doseur sur ce substrat, celui-ci se trouve à une distance du dispositif doseur de 20 cm au maximum.
